# EUROPEAN PATENT SPECIFICATION

(11) **EP 2 498 845 B1**
(45) Date of publication and mention of the grant of the patent: **12.04.2017**
(21) Application number: 10782707.3
(22) Date of filing: 02.11.2010
(51) Int. Cl.: A61M 15/00

(54) **DRUG DELIVERY APPARATUS AND METHOD**
VORRICHTUNG UND VERFAHREN ZUR WIRKSTOFFFREISETZUNG
APPAREIL D'ADMINISTRATION DE MÉDICAMENT ET PROCÉDÉ ASSOCIÉ

(30) Priority: 11.11.2009 EP 09175628
(43) Date of publication of application: 19.09.2012
(73) Proprietor: Koninklijke Philips N.V., 5656 AE Eindhoven (NL)
(72) Inventor: DENYER, Jonathan, Stanley, Harold, NL-5656 AE Eindhoven (NL); DYCHE, Anthony, NL-5656 AE Eindhoven (NL); LEPPARD, Michael, James, Robbert, NL-5656 AE Eindhoven (NL); PETHERBRIDGE, Ian, Thomas, NL-5656 AE Eindhoven (NL); SCHIPPER, Alphonsus, Tarcisius, Jozef, Maria, NL-5656 AE Eindhoven (NL)
(74) Representative: van Velzen, Maaike Mathilde
(86) International application number: PCT/IB2010/054951
(87) International publication number: WO 2011/058477

(56) References cited:
- US-A1- 2004 094 152
- US-A1- 2004 158 349
- US-A1- 2004 231 667
- US-A1- 2007 076 067
- US-A1- 2007 163 583
- US-A1- 2008 060 641

## Description

### FIELD OF THE INVENTION

The invention relates to a drug delivery apparatus and method and in particular to a nebulizer used for drug delivery, and a method of operating such a nebulizer.

### BACKGROUND OF THE INVENTION

Nebulizers, or atomizers as they are sometimes called, are devices that generate a fine spray or aerosol, usually of liquid. A particularly useful application for nebulizers is to provide a fine spray containing a dissolved or a suspended particulate drug for administration to a patient by inhalation.

Piezo-mesh based nebulizers are commonly used to generate aerosols in such drug delivery apparatus, whereby a piezoelectric element vibrates a mesh to produce the fine aerosol spray. In particular, droplets dispensed on the mesh are vibrated by the piezoelectric element to create the spray. There are two principle designs in such piezo-mesh based nebulizers. United States patents US5938117 and US6983747 disclose one type of design whereby the piezoelectric element is bonded to a mesh element, whereas US6651650 and US6405934 disclose designs whereby the mesh element is separate from the piezoelectric element. An advantage of having the mesh element separate from the piezoelectric element is that the mesh element is cheaper to manufacture and so can be replaced more frequently.

However, a common disadvantage of all mesh based nebulizers is that a user is required to clean the mesh after use, otherwise the mesh holes may become blocked. There are in the region 5000 2µm holes in a typical mesh, and these can easily become blocked by particulates in the environment or from salt crystals (i.e. because the drugs are often saline based).

The cleaning method is normally to wash the mesh in warm soapy water for about five minutes, and then rinse and dry the mesh. This process can take as much time as the drug treatment itself, and is therefore a significant burden on the patient. Hence, over time, even with good cleaning the performance of the mesh will deteriorate as more holes become blocked - this may typically happen over a number of weeks. Once the mesh becomes blocked it is very difficult to clean the mesh and remove a particulate which is lodged in a hole, so it is necessary to replace the mesh. Although meshes are designed to last for up to twelve months, they typically have to be replaced every three months, or even on a monthly basis for some patients due to poor cleaning.

Such drug delivery apparatus also typically comprise one or more interchangeable parts, for example interchangeable mouthpieces, interchangeable plunger assemblies and/or interchangeable medication chambers. These interchangeable parts enable a nebulizer to be adapted or customized to best suit the needs of a particular patient. For example, different mouthpieces can be selected depending on the volume of inhalation preferred by a particular user, while different medication chambers can be selected to provide different volumes depending on the drug to be delivered.

A disadvantage of having such interchangeable parts is that the drug delivery apparatus must know which particular part from a set of interchangeable parts is fitted at any particular time, so that the drug delivery apparatus is able to control the delivery of the drug accordingly. The prior art documents US 2007/163583 A1 and US 2008/060641 A1 only disclose the use of data carriers with medication chambers, where the primary aim of such data carriers is to check the type of drug contained in the medication chamber, the correct dose for that drug, or that the drug is from an authorized source or distributor. It is an aim of the present invention to provide a drug delivery system that helps alleviate or reduce one or more of the disadvantages mentioned above.

### SUMMARY OF THE INVENTION

According to a first aspect of the invention there is provided a nebulizer comprising a removable component comprising a data carrier, and a data reader for communicating with the data carrier of the removable component.

The removable component may be one of a set of associated removable components. The data reader thereby enables the nebulizer to determine which removable component from the set of removable components is attached to the nebulizer, thus enabling the operation of the nebulizer to be controlled accordingly.

According to another aspect of the invention, there is provided a method of operating a nebulizer, the method comprising the steps of receiving information from a data carrier associated with a removable component of the nebulizer, and controlling the operation of the nebulizer based on the information received from the data carrier.

According to another aspect of the invention there is provided a mesh assembly for use in a nebulizer, the mesh assembly comprising a data carrier for communicating, in use, with a data reader provided in the nebulizer.

According to another aspect of the invention there is provided a mouthpiece for use with a drug delivery apparatus, the mouthpiece comprising a data carrier for communicating, in use, with a data reader provided in the nebulizer.

According to another aspect of the invention there is provided a medication chamber for use with a drug delivery apparatus, the medication chamber comprising a data carrier for communicating, in use, with a data reader provided in the nebulizer.

According to another aspect of the invention there is provided a metering chamber for use with a drug delivery apparatus, the metering chamber comprising a data carrier for communicating, in use, with a data reader provided in the nebulizer. The data carrier provides information relating to the metered drug dose to the nebulizer.

According to another aspect of the invention there is provided a plunger assembly for use with a drug delivery apparatus, the plunger assembly comprising a data carrier for communicating, in use, with a data reader provided in the nebulizer.

### BRIEF DESCRIPTION OF THE DRAWINGS

For a better understanding of the present invention, and to show more clearly how it may be carried into effect, reference will now be made, by way of example only, to the following drawings in which:
Fig. 1 shows a nebulizer according to an embodiment of the present invention;
Figs. 2a to 2c show a mesh assembly of a nebulizer in greater detail, according to another embodiment of the invention;
Figs. 3a to 3c show a mesh assembly of a nebulizer in greater detail, according to another embodiment of the invention;
Fig. 4 shows a flow chart describing the steps performed by one embodiment of the present invention;
Fig. 5 shows a nebulizer according to another embodiment of the present invention;
Fig. 6 shows a flow chart describing the steps performed by an embodiment of the present invention;
Fig. 7 shows a flow chart describing the steps performed by an embodiment of the present invention; and
Fig. 8 shows a nebulizer according to another embodiment of the present invention.

### DETAILED DESCRIPTION OF THE EMBODIMENTS

The embodiments below will be described in relation to piezo-mesh type drug delivery apparatus. It is noted, however, that some embodiments are not necessarily limited to such piezo-mesh drug delivery apparatus, for example the embodiments relating to nebulizers having interchangeable components such as mouthpieces and medication chambers. Also, it is noted that the term nebulizer can be used interchangeably with the term drug delivery apparatus or atomizer, and is intended to cover other forms and designs of nebulizer other than the specific type of nebulizer described below and illustrated in the Figures.

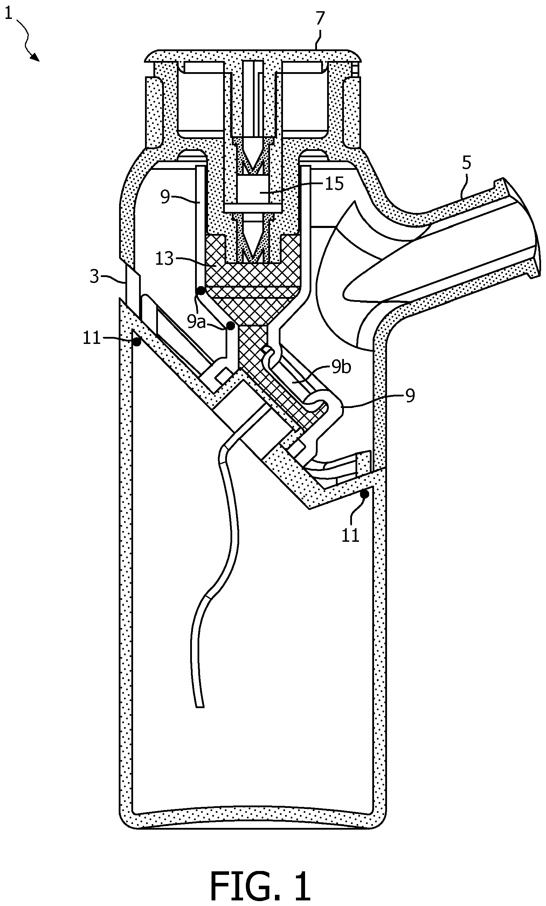

Figure 1 shows a nebulizer 1 according to an embodiment of the present invention. The nebulizer 1 comprises a body 3 which receives one or more removable components (i.e. interchangeable parts), such as a mouthpiece 5, a plunger assembly 7 and a mesh assembly 9. The mesh assembly 9 comprises a mesh 9b, which is vibrated by a piezoelectric element to generate a fine spray or aerosol. According to one aspect of the invention the mesh assembly 9 also comprises a data carrier 9a. The data carrier 9a communicates with a data reader 11 mounted in the nebulizer 1, for example in the body of the nebulizer. In this particular embodiment the data carrier 9a comprises an RFID tag and the data reader 11 an antenna, each comprising a coil (with Figure 1 showing a cross section of each coil loop, the coil planes being perpendicular to the plane of the image). The operation of an RFID tag and antenna will be familiar to those skilled in the art. The invention is intended to cover the use of any type of RFID tagging system including, but not limited to, passive RFID tags (i.e. which are powered from the energy received from the associated antenna) or active RFID tags (i.e. which are self-powered). As discussed later in the application, other forms of data carrier and data reader are also envisaged, without departing from the scope of the invention.

The data carrier 9a associated with the mesh 9b provides information relating to the mesh 9b to the nebulizer 1, as will be described later in the application.

In the embodiment of Figure 1 it can be seen that the body of the mesh assembly 9 also forms a medication chamber 13. As such, the data carrier 9a can also be used to provide information relating to the medication chamber 13 to the nebulizer 1. The plunger assembly 7 comprises a medication metering chamber 15. The medication metering chamber 15 is arranged to feed the drug to be nebulized to the nebulation device for nebulization, while the medication chamber 13 is arranged to hold and retain any of the drug in excess of the volume held in the medication metering chamber 15. This allows a unit dose (i.e. vial) of a drug to be nebulized to be poured into a reservoir, but only the metered volume of the medication metering chamber 15 to be nebulized during treatment, with the remainder or excess of the drug being retained in the medication chamber 13. Further details of the medication metering chamber 15 and its operation can be found in United States patent US2003/0146300A1.

Although the embodiment of Figure 1 shows the medication chamber 13 being formed using the mesh assembly 9, it is noted that the medication chamber can be formed as a separate physical entity, in which case the medication chamber could have a separate data carrier for providing information relating to the medication chamber to the nebulizer (i.e. a separate data carrier from that associated with the mesh).

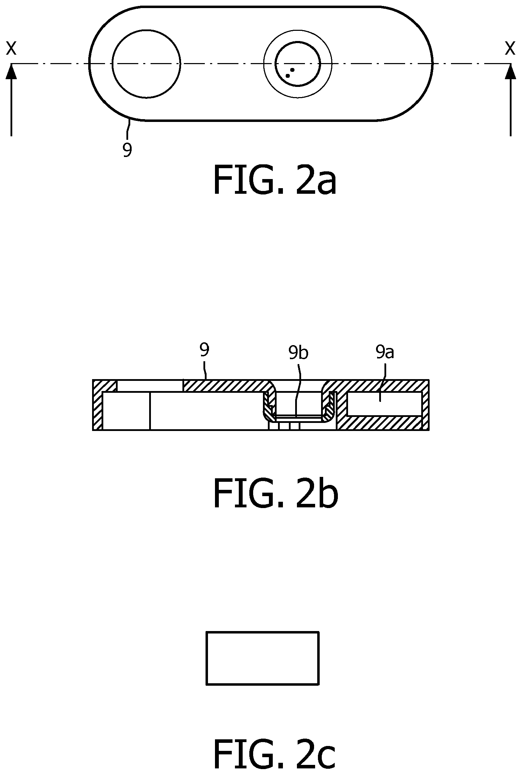

Figures 2a to 2c show in greater detail a mesh assembly 9 that is configured differently to the mesh assembly 9 of Figure 1. Figure 2a shows a plan view of the mesh assembly 9. Figure 2b shows a side sectional view through section X-X of Figure 2a. The mesh assembly 9 of this particular embodiment is configured such that the data carrier 9a and mesh 9b lie in the same plane, i.e. co-planar. This has the advantage of enabling the mesh assembly 9 to be attached to a drug pack. Figure 2c shows an end view of the mesh assembly 9. It will be appreciated that in this particular embodiment the mesh assembly 9 does not form a medication chamber 13 as shown in Figure 1, which means that the nebulizer 1 would require a separate medication chamber (and possibly a separate data carrier associated with such a medication chamber).

In a further embodiment, not shown in Figs. 2a-2c, the mesh assembly 9 may comprise the mesh 9b, the metering chamber and the data carrier 9a. The data carrier may provide information on the mesh and the metered dose to the nebulizer which information may be used by a clinician for example for error checking. For example when the total time of a treatment for a patient is longer then would be expected based on a flow rate of the nebulizer and information from the data carrier on the drug dose and metering chamber volume the clinician may conclude that the mesh needs to be replaced.

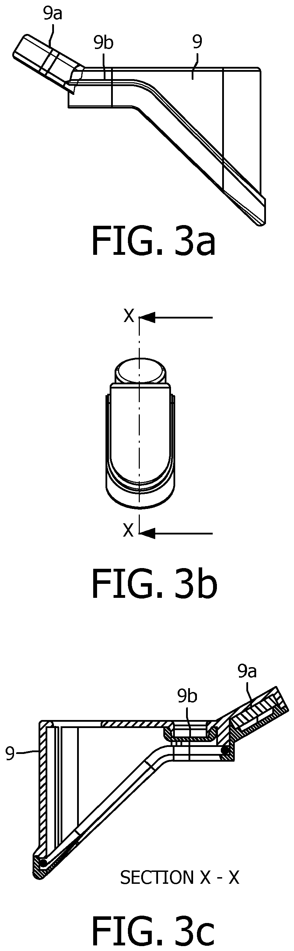

Figures 3a to 3c show a further alternative configuration of a mesh assembly 9. Figure 3a shows a side elevation of the mesh assembly 9, which comprises the data carrier 9a and the mesh 9b. Figure 3b shows an end elevation, while Figure 3c shows a sectional view through section X-X of Figure 3b. According to this particular embodiment the data carrier 9a lies in a different plane to that of the mesh 9b, which may be desirable is certain applications, for example to enable the data carrier 9a to be located more closely to a corresponding data reader 11 of the nebulizer. It will be appreciated that various configurations for mounting the data carrier 9a and mesh 9b are possible, depending on the particular application and the type of nebulizer being used.

The data carrier 9a associated with a mesh 9b of a mesh assembly 9 contains information pertaining to the mesh 9b, which can be read by the data reader 11 of the nebulizer 1. For example, the data carrier 9a may be used to identify the type of mesh 9b being used. The data carrier 9a may contain information on the intended use or lifespan of the mesh 9b, for example how many times the mesh 9b should be used before being replaced. Preferably the whole mesh assembly 9 (including the data carrier 9a and mesh 9b) is replaced after a predetermined number of uses.

Alternatively, if the data carrier 9a and the mesh 9b are detachably connected to the body of the mesh assembly 9, then the data carrier 9a and mesh 9b may be replaced independently of the main body of the mesh assembly 9. For example, the user may purchase a data carrier 9a and a mesh 9b which are replaced as a set, by fitting a new data carrier 9a and a new mesh 9b to the existing body of the mesh assembly 9.

The information received by the data reader 11 may be used by the nebulizer for a number of purposes. According to one embodiment the information received by the data reader 11 can be used to count the number of times a particular mesh 9b has been used, and then prevent the nebulizer from being operated after the mesh 9b has been used a predetermined number of times. The mesh 9b can therefore be prevented from being used in the drug delivery apparatus when its intended lifespan has expired. Alternatively, or in addition, the nebulizer may be configured to provide some form of indication or warning to the user once the intended lifespan has expired, i.e. rather than preventing the nebulizer from being used entirely. This type of indication or warning encourages the user to replace the mesh, but without preventing the nebulizer from being used.

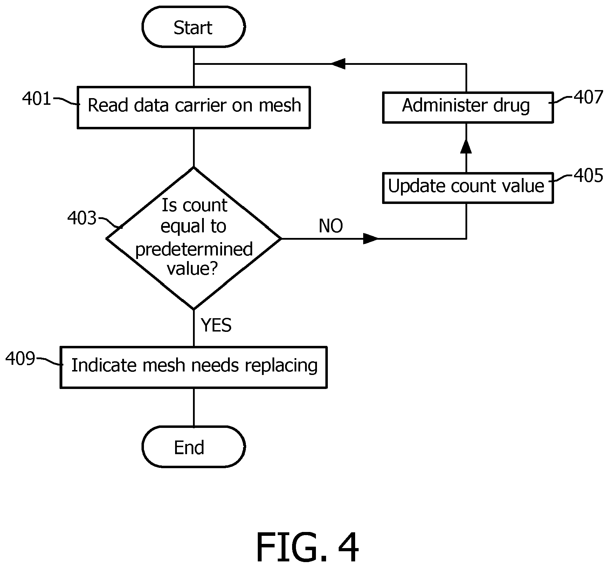

Figure 4 illustrates the steps that may be performed in a nebulizer having a mesh that has an associated data carrier, as described above in Figures 1 to 3. The steps shown in Figure 4 may be performed as part of a drug delivery operation, for example in response to a user triggering a drug delivery operation. In step 401 the nebulizer reads the data carrier associated with the mesh. In step 403 the nebulizer reads a data field on the data carrier to ascertain a count value relating to the use of the mesh, and determines whether the count value is equal to a predetermined value. For example, if a particular mesh is intended to be used 255 times and is supplied with a count value of 255, which is decremented after each use, step 403 may involve checking whether the count value has reached zero. Alternatively, if the mesh is supplied with a count value of zero which is incremented after each use, step 403 may involve checking whether the count value has reached a predetermined value, i.e. 255 in this particular example.

If it is determined in step 403 that the count value is not equal to a predetermined value, then in step 405 the count value is updated (i.e. incremented or decremented), and the nebulizer operated to deliver a drug, step 407.

If it is determined in step 403 that the count value is equal to a predetermined value, thereby indicating that the mesh has been used a predetermined number of times, the nebulizer indicates in step 409 that the mesh requires replacing. This may involve disabling the nebulizer such that the mesh can no longer be used.

As mentioned above, as an alternative to preventing the nebulizer from being operated after the mesh has been used a predetermined number of times, the nebulizer may be configured instead (or in addition) to provide a warning to the user that the mesh should be replaced. For example, the nebulizer may be configured to provide a visual and/or audible warning when the mesh has reached its expected lifespan.

The nebulizer may also be configured to provide such a warning at a predetermined interval prior to the mesh coming to the end of its life, thereby warning the user to purchase a new mesh.

Preferably the updated count value shown in step 405 is stored on the data carrier 9a associated with the mesh 9b. As such, the data reader 11 acts as a data writer in addition to a data reader. In other words, the data reader 11 (for example an antenna) is adapted to transmit data to the data carrier 9a, as well as reading data from the data carrier 9a. In such an embodiment the data carrier will include, for example, an electrically erasable memory as will be familiar to those skilled in the art, such as an Electrically Erasable Programmable Read Only Memory (EEPROM). Other forms of data carriers that are capable of storing and updating a count value are also intended to be embraced by the present invention.

As an alternative to the above, the updated count value may be stored in the nebulizer itself. With such an embodiment the data carrier 9a may have a simpler form of memory device, such a Read Only Memory (ROM) which is programmed once during manufacture with a count value corresponding to the intended lifespan of the mesh. However, the former method has the advantage of retaining the count value with the device that is actually being monitored, which provides a more secure application.

It is noted that the data carrier 9a and data reader 11 can be realized in alternative ways to using an RFID tag and an antenna. For example, other identifying means such as a barcode, DX or serial interface can be used to communicate information between the mesh and the nebulizer. However, using an RFID tag and an antenna has the advantage of not requiring any interconnecting electrical contacts, which could otherwise become degraded in the type of environment found within a nebulizer device.

In addition to the data carrier 9a being used to store information relating to the use of the corresponding mesh 9b, it is noted that the data carrier 9a may also be used to store other information, such as information relating to the drug being dispensed. In other words, since the data carrier 9a is used to store information about the use of the nebulizer, the data carrier 9a may also be used to store other information relating to such use, including the number of drug vials to be dispensed. Thus, if a mesh of the nebulizer is replaced at the same time as the drug container, the data carrier 9a of the mesh can be used to indicate when the drug container needs to be replaced. Also, as indicated above in Figure 1, if the mesh assembly 9 forms part of the medication chamber, then the data carrier 9a may also store information relating to the medication chamber.

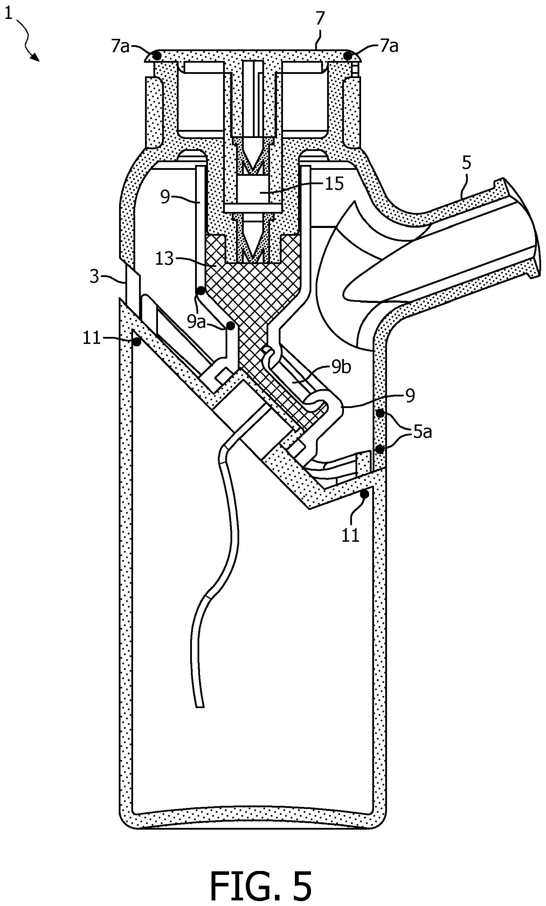

Figure 5 shows a nebulizer 1 according to another embodiment of the present invention. The nebulizer 1 comprises a body 3 for receiving one or more removable components, such as a mouthpiece 5, a plunger assembly 7 and a mesh assembly 9. As with Figure 1, the mesh assembly 9 forms a medication chamber 13 (although it is noted that these could be separate physical components, without departing from the scope of the invention). The plunger assembly 7 comprises a medication metering chamber 15. According to this embodiment the mesh assembly 9 comprises a first data carrier 9a, the mouthpiece 5 comprises a second data carrier 5a, and the plunger assembly 7 comprises a third data carrier 7a. The first data carrier 9a associated with the mesh assembly 9 also doubles as a medication chamber data carrier for this particular embodiment. Each of the data carriers 5a, 7a and 9a communicate with a data reader 11 mounted in the body 3 of the nebulizer 1.

One or more of the removable components 5, 7 or 9 may be a removable component associated with a set of such removable components (i.e. a form of interchangeable part selected from a set of such interchangeable parts). For example, the removable mouthpiece 5 can be from a set of different mouthpieces that may be fitted to the nebulizer. The nebulizer comprises control means for controlling the operation of the nebulizer depending on the particular removable component that is attached to the nebulizer at a given time.

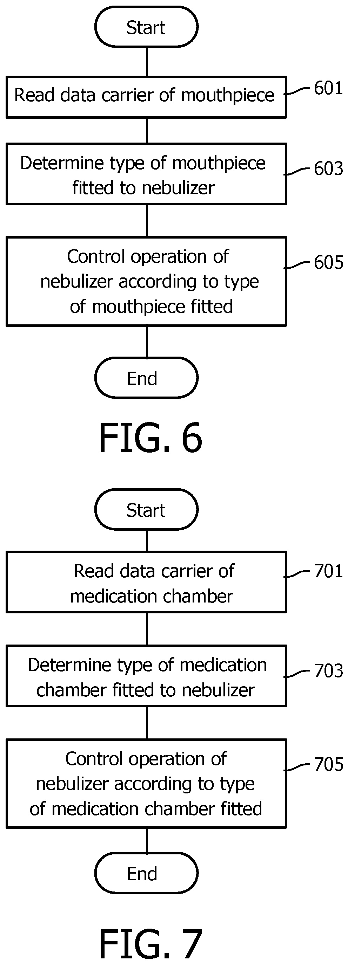

For example, Figure 6 illustrates the steps that may be performed in a nebulizer depending on the type of mouthpiece fitted to the nebulizer. In step 601 the nebulizer reads the data carrier 5a of the mouthpiece 5. In step 603 the nebulizer determines the type of mouthpiece 5 that is fitted to the nebulizer using the information gathered from the data carrier 5a. Then, in step 605, the nebulizer adjusts or controls the operation of the nebulizer according to which type of mouthpiece 5 is fitted to the nebulizer.

The mouthpiece 5 is part of a set of associated mouthpieces that may be used with the nebulizer, for example depending on the particular preference of the user, or which is best suited for delivery of a particular type of drug.

For example, patients could be supplied with two or more mouthpieces with varying resistances to suit their personal preference, such as a first mouthpiece giving a high resistance of about 14-18 liters/min , a second mouthpiece giving a low resistance of about 24-36 liters/min, and a third mouthpiece giving a resistance of about 40 - 60 liters/min. The control software in the nebulizer needs to know which mouthpiece is fitted in order to enable the nebulizer to operate correctly, and the data carrier 5a mounted on the mouthpiece can be used to store such information during manufacture.

Figure 7 illustrates the steps that may be performed in a nebulizer depending on the type of medication chamber 13 fitted to the nebulizer. In step 701 the nebulizer reads the data carrier of the medication chamber 13 (which would be the data carrier 9a in Figures 1 and 5 due to the medication chamber 13 being formed from the mesh assembly 9 - a separate data carrier being provided when the medication chamber is formed from a separate physical entity to that of the mesh assembly). In step 703 the nebulizer determines the type of medication chamber that is fitted to the nebulizer using the information gathered from the data carrier 9a. Then, in step 705, the nebulizer adjusts or controls the operation of the nebulizer according to which type of medication chamber is fitted to the nebulizer.

The medication chamber 13 is part of a set of associated medication chambers 13 that may be used with the nebulizer, for example depending on which particular medication chamber is best suited for delivery of a particular type of drug.

In some applications more than one volume of drug can be delivered from the same drug pack by using different medication chambers. In such applications the drug dose information on the data carrier 9a associated with the mesh 9b may not be sufficient for the nebulizer software to accurately determine the drug dose. This is improved by adding a data carrier to the medication chamber 13 as described above (or to the metering system) for the medication chamber 13 to uniquely identify the drug dose which has been placed in the device.

The nebulizer may be configured to perform other features based on the information detected by the data reader 11. For example, the nebulizer can be configured to only start a treatment once a predetermined set of components are detected as being attached to the nebulizer, such as when the mesh assembly 9, mouthpiece 5, plunger assembly 7 and medication chamber 13 have been detected as being present.

Although the embodiment of Figure 5 shows a single data reader 11 being provided for communicating with each of the data carriers 5a, 7a and 9a relating to the mouthpiece 5, plunger assembly 7 and mesh assembly 9 respectively, it is noted that the nebulizer may comprise one or more data readers 11 for communicating with one or more data carriers. As with the embodiment of Figure 1, the data carriers 5a, 7a and 9a may be RFID tags and the data reader(s) 11 an antenna, although other forms of communication means are also intended to be embraced by the present invention.

The range of the RFID system may be set so that only assembled components and their associated data carriers are detected by the data reader(s) 11. This has the advantage that RFID tagged components located nearby, but not affixed to the nebulizer, are not detected by the data reader(s) 11.

This may be achieved by designing and positioning the data reader 11 and associated data carriers 5a, 7a, 9a so that the field of operation is limited to the shortest range required, and positioning the data carriers close to the data reader.

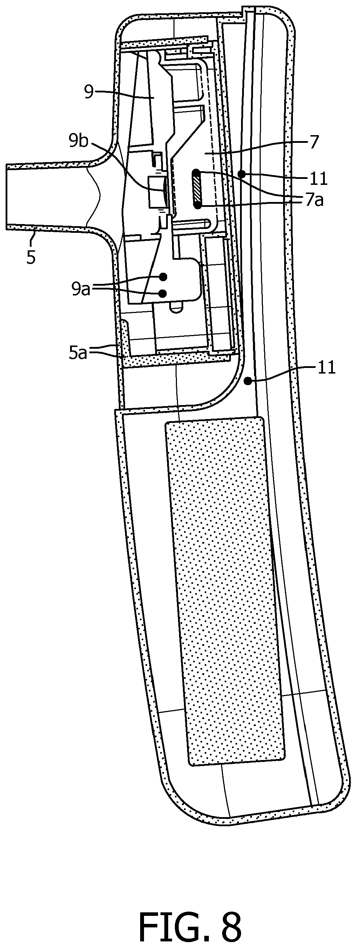

Figure 8 shows an alternative embodiment, whereby the configuration of the nebulizer is adapted to enable the data carrier 7a associated with the plunger assembly 7 to be brought into closer proximity with the data reader 11, thereby enabling the range of the RFID system to be reduced, and hence reducing the possibility of interference from other devices. As with Figure 5, the nebulizer comprises a mouthpiece 5 having an associated data carrier 5a, a plunger assembly 7 having an associated data carrier 7a, and a mesh assembly 9 having an associated data carrier 9a.

The nebulizer described in the embodiments above may also adopt one or more other strategies to help reduce or prevent detection of unwanted items by the radio frequency tagging system. For example, shielding may be provided on the side of the data reader 11 that is opposite to the side where the data carriers 5a, 7a and 9a are positioned.

In the embodiments described above the RFID data transmission could be interrupted by an external RF source such as the piezoelectric element used to vibrate the mesh, or by mobile phones that are within close proximity. However, the former does not have a degrading effect on the operation of the RFID system, since the RFID tags are normally read prior to the drug delivery operation itself, i.e. prior to the piezoelectric circuit being activated, and hence prior to such interference being present. Interference from other sources such as mobile phones can be overcome using data correction techniques commonly found in wireless technology, as will be familiar to a person skilled in the art.

The RFID system may consist of an integrated circuit with a copper coil, which can be encapsulated within a thin plastic film. This may typically comprise a product that is about 10mm in diameter and about 1-2 mm tick. It will be appreciated, however, that other dimensions can also be used depending on the particular nebulizer, and without departing from the scope of the invention.

The RFID tag may be integrated into a plastic housing either mechanically, by potting or over molding to produce a hermetically sealed assembly as shown in Figures 2a to 2c or 3a to 3c. This plastic housing can also incorporate the mesh required for aerosol generation which is typically 10 mm diameter and 0.025-0.5 mm thick, this can also be mounted mechanically or over molded. This generates an assembly typically 35 x 20 mm and 2-3 mm thick or if the components are mounted co axially 25mm diameter and 2- 3 mm thick. It will be appreciated, however, that other mounting arrangements and configurations may be used without departing from the scope of the invention.

It is noted that data carriers may also be added to other removable components of the nebulizer, such as ID cards or labels.

Table 1 below illustrates some parameters of the reader coil and tag coils that may be used, for example, in a nebulizer according to an embodiment of the invention.

**Table 1**

| | Diameter [cm] | Windings | Distance [cm] | Offset [cm] | Alpha |
|---|---|---|---|---|---|
| Reader coil | 4.0 | 2 | 0.0 | | 0 |
| Mesh coil | 1.0 | 18 | 1.3 | 1.4 | 0 |
| Mouthpiece coil | 1.0 | 18 | 1.5 | 1.5 | 45 |
| Plunger | 1.0 | 18 | 5.6 | 3.0 | 45 |
| ID card coil | | | 2.0 | 0.0 | 0 |
| Label coil | | | 2.0 | 0.0 | 0 |

- Diameter:: Diameter of coil
- Windings:: Number of windings
- Offset:: Offset from center of reader coil
- Alpha:: Angle from plane parallel to reader coil plan

The antenna may be a dedicated antenna, which can be tuned to 13.56MHz, for example. The antenna may comprise four circular windings with a diameter of 40mm.

It is also noted that the RFID tag associated with the mesh may contain a number of fields related to the mesh and/or the drug and/or the delivery system. For example, a "Vial Count Remaining" field may be used to determine when the tag can no longer be used as it is decremented by one each time a treatment is delivered.

Although the embodiments described above have been made in relation to interchangeable mouthpieces, medication chambers or meshes, it will be appreciated that the invention is equally applicable to any other interchangeable part of a drug delivery apparatus, and in particular interchangeable parts from a set of such interchangeable parts.

The term "removable component" is intended to embrace a part or component of the nebulizer, at least part of which contributes to the actual functioning of the nebulizer, rather than a non-functioning part such as the actual drug to be dispensed. In other words, at least part of the removable component is a physical part of the actual nebulizer, which contributes to the physical working of the nebulizer.

Furthermore, although the embodiments of Figures 1 and 5 show a nebulizer having a removable mesh assembly, a removable mouthpiece, a removable plunger assembly and removable medication chamber, it will be appreciated that not all of these components need necessarily be removable, or be from sets of interchangeable parts. For example, in the embodiment of Figure 1 the mouthpiece and medication chamber may be fixed components, rather than removable components.

It will be appreciated from the above that RFID tags are ideally suited to the described embodiments, as they require no electrical contacts that may be affected by the saline based drugs often used in the drug products to be inhaled, which would otherwise affect alternative embodiments such as DX or serial interfaces that require electrical contacts. RFID tags are also more suitable than bar codes as the vial count can be decremented in the tag itself after each treatment which is more secure than reading a bar code and decrementing the count within the delivery system itself. However, as mentioned above, the invention is not limited to just RFID tags, and that other identification systems can be used without departing from the scope of the invention.

It is also noted that the invention can be used with piezo-mesh type nebulizers in which the piezoelectric element and the mesh are bonded together, or with piezo-mesh type nebulizers in which the piezoelectric element and mesh are formed separately.

It should be noted that the above-mentioned embodiments illustrate rather than limit the invention, and that those skilled in the art will be able to design many alternative embodiments without departing from the scope of the appended claims. The word "comprising" does not exclude the presence of elements or steps other than those listed in a claim, "a" or "an" does not exclude a plurality, and a single processor or other unit may fulfill the functions of several units recited in the claims. Any reference signs in the claims shall not be construed so as to limit their scope.

## Claims

1. A nebulizer (1) comprising:
a removable component (9) comprising a data carrier (9a); and
a data reader (11) for communicating with the data carrier (9a) of the removable component (9);
wherein the removable component comprises a mesh assembly (9); and
wherein the nebulizer further comprises control means for controlling the operation of the nebulizer (1) based on information provided by the data carrier (9a).

2. A nebulizer (1) as claimed in claim 1, wherein the nebulizer comprises at least one further removable component (5, 7, 13) comprising a data carrier (5a, 7a).

3. A nebulizer (1) as claimed in claim 1 or 2, wherein the or each removable component (5, 7, 9, 13) is one of a set of associated removable components.

4. A nebulizer (1) as claimed in claim 3, wherein the data reader (11) is adapted to identify a particular removable component (5, 7, 9, 13) from its associated set of removable components using information contained in the data carrier (5a, 7a, 9a) of the removable component (5, 7, 9, 13).

5. A nebulizer (1) as claimed in any one of claims 2 to 4, wherein the control means is adapted to control the operation of the nebulizer (1) based on information provided by the at least one further data carrier (5a, 7a, 9a).

6. A nebulizer (1) as claimed in claim 5, wherein the control means is configured such that the operation of the nebulizer is only possible once a predetermined number of removable components (5, 7, 9, 13) are detected as being attached to the nebulizer (1).

7. A nebulizer (1) as claimed in claim 1, wherein the data carrier (9a) stores a count value relating to the use of the mesh assembly (9), and wherein the control means is adapted to preventing a mesh (9b) from being used in the nebulizer after a predetermined number of uses.

8. A nebulizer (1) as claimed in any one of the preceding claims, wherein the data carrier (5a, 7a, 9a) and the data reader (11) comprise a radio frequency identification (RFID) tagging system.

9. A nebulizer (1) as claimed in claim 8, wherein the data carrier (5a, 7a, 9a) comprises an RFID tag and the data reader (11) comprises an antenna.

10. A nebulizer (1) as claimed in claim 8 or 9, wherein the range of the RFID tagging system is restricted such that the antenna (11) is suited to communicate with RFID tags (5a, 7a, 9a) mounted in removable components (5, 7, 9, 13) attached to the nebulizer.

11. A nebulizer (1) as claimed in any one of claims 2 to 9, wherein the at least one further removable component (5, 7, 9, 13) is one of a mouthpiece (5), a plunger assembly (7) or a medication chamber (13).

12. A mesh assembly (9) for use in a nebulizer, the mesh assembly (9) comprising a data carrier (9a) for communicating, in use, with a data reader (11) provided in the nebulizer;
wherein the data carrier (9a) is adapted to store information for controlling the operation of the nebulizer.

13. A mesh assembly (9) as claimed in claim 12, wherein the data carrier (9a) is adapted to store information for preventing a mesh (9b) from being used in a nebulizer after a predetermined number of uses.

14. A mesh assembly (9) as claimed in claim 13, wherein the data carrier (9a) stores a count value, the count value being incremented or decremented after each use of the mesh.

15. A method of operating a nebulizer (1), the method comprising the steps of:
receiving information from a data carrier (5a, 7a, 9a) associated with a removable component (5, 7, 9, 13) of the nebulizer; and
controlling the operation of the nebulizer based on the information received from the data carrier (5a, 7a, 9a);
wherein the removable component comprises a mesh assembly.

## Patentansprüche

1. Vernebler (1), umfassend:
eine entfernbare Komponente (9) mit einem Datenträger (9a); sowie
einen Datenleser (11) zur Kommunikation mit dem Datenträger (9a) der entfernbaren Komponente (9);
wobei die entfernbare Komponente eine Mesh-Anordnung (9) umfasst; und
wobei der Vernebler weiterhin Steuermittel umfasst, um den Betrieb des Verneblers (1) aufgrund von, durch den Datenträger (9a) bereitgestellten Informationen zu steuern.

2. Vernebler (1) nach Anspruch 1, wobei der Vernebler mindestens eine weitere entfernbare Komponente (5, 7, 13) mit einem Datenträger (5a, 7a) umfasst.

3. Vernebler (1) nach Anspruch 1 oder 2, wobei die oder jede entfernbare Komponente (5, 7, 9, 13) eine aus einem Satz von zugeordneten, entfernbaren Komponenten ist.

4. Vernebler (1) nach Anspruch 3, wobei der Datenleser (11) so eingerichtet ist, dass er eine bestimmte entfernbare Komponente (5, 7, 9, 13) aus ihrem zugeordneten Satz von entfernbaren Komponenten unter Verwendung von in dem Datenträger (5a, 7a, 9a) der entfernbaren Komponente (5, 7, 9, 13) enthaltenen Informationen identifiziert.

5. Vernebler (1) nach einem der Ansprüche 2 bis 4, wobei das Steuermittel so eingerichtet ist, dass es den Betrieb des Verneblers (1) aufgrund von, durch den mindestens einen weiteren Datenträger (5a, 7a, 9a) bereitgestellten Informationen steuert.

6. Vernebler (1) nach Anspruch 5, wobei das Steuermittel so konfiguriert ist, dass der Betrieb des Verneblers nur dann möglich ist, wenn eine vorher festgelegte Anzahl von entfernbaren Komponenten (5, 7, 9, 13) als an den Vernebler (1) angebracht ermittelt wird.

7. Vernebler (1) nach Anspruch 1, wobei der Datenträger (9a) einen auf die Verwendung der Mesh-Anordnung (9) bezogenen Zählwert speichert, und wobei das Steuermittel so eingerichtet ist, dass es verhindert, dass eine Mesh (9b) nach einer vorher festgelegten Anzahl von Verwendungen weiter verwendet wird.

8. Vernebler (1) nach einem der vorangegangenen Ansprüche, wobei der Datenträger (5a, 7a, 9a) und der Datenleser (11) ein RFID- (Radio Frequency Identification) Tagging-System umfassen.

9. Vernebler (1) nach Anspruch 8, wobei der Datenträger (5a, 7a, 9a) ein RFID-Tag umfasst und der Datenleser (11) eine Antenne umfasst.

10. Vernebler (1) nach Anspruch 8 oder 9, wobei der Bereich des RFID-Tagging-Systems so beschränkt ist, dass die Antenne (11) dafür geeignet ist, mit RFID-Tags (6a, 7a, 9a), die in an dem Vernebler angebrachten, entfernbaren Komponenten (5, 7, 9, 13) befestigt sind, zu kommunizieren.

11. Vernebler (1) nach einem der Ansprüche 2 bis 9, wobei die mindestens eine weitere entfernbare Komponente (5, 7, 9, 13) ein Mundstück (5), eine Kolbenanordnung (7) oder eine Medikationskammer (13) ist.

12. Mesh-Anordnung (9) zum Einsatz in einem Vernebler, wobei die Mesh-Anordnung (9) einen Datenträger (9a) umfasst, um bei Betrieb mit einem in dem Vernebler vorgesehenen Datenleser (11) zu kommunizieren,
wobei der Datenträger (9a) so eingerichtet ist, dass er Informationen zur Steuerung des Betriebs des Verneblers speichert.

13. Mesh-Anordnung (9) nach Anspruch 12, wobei der Datenträger (9a) so eingerichtet ist, dass er Informationen speichert, um zu verhindern, dass eine Mesh (9b) nach einer vorher festgelegten Anzahl von Verwendungen in einem Vernebler weiter verwendet wird.

14. Mesh-Anordnung (9) nach Anspruch 13, wobei der Datenträger (9a) einen Zählwert speichert, wobei der Zählwert nach jeder Verwendung der Mesh inkrementiert oder dekrementiert wird.

15. Verfahren zum Betreiben eines Verneblers (1), wobei das Verfahren die folgenden Schritte umfasst, wonach:
Informationen von einem Datenträger (5a, 7a, 9a) empfangen werden, der einer entfernbaren Komponente (5, 7, 9, 13) des Verneblers zugeordnet ist; und
der Betrieb des Verneblers aufgrund der von dem Datenträger (5a, 7a, 9a) empfangenen Informationen gesteuert wird;
wobei die entfernbare Komponente eine Mesh-Anordnung umfasst.

## Revendications

1. Nébuliseur (1) comprenant :
un composant amovible (9) comprenant un support de données (9a) ; et
un lecteur de données (11) pour communiquer avec le support de données (9a) du composant amovible (9) ;
dans lequel le composant amovible comprend un ensemble grille (9) ; et
dans lequel le nébuliseur comprend en outre un moyen de commande pour commander le fonctionnement du nébuliseur (1) d'après des informations fournies par le support de données (9a).

2. Nébuliseur (1) selon la revendication 1, dans lequel le nébuliseur comprend au moins un composant amovible (5, 7, 13) supplémentaire comprenant un support de données (5a, 7a).

3. Nébuliseur (1) selon la revendication 1 ou 2, dans lequel le ou chaque composant amovible (5, 7, 9, 13) est l'un d'un jeu de composants amovibles associés.

4. Nébuliseur (1) selon la revendication 3, dans lequel le lecteur de données (11) est adapté pour identifier un composant amovible (5, 7, 9, 13) particulier parmi son jeu associé de composants amovibles à l'aide d'informations contenues dans le support de données (5a, 7a, 9a) du composant amovible (5, 7, 9, 13).

5. Nébuliseur (1) selon l'une quelconque des revendications 2 à 4, dans lequel le moyen de commande est adapté pour commander le fonctionnement du nébuliseur (1) d'après des informations fournies par l'au moins un support de données (5a, 7a, 9a) supplémentaire.

6. Nébuliseur (1) selon la revendication 5, dans lequel le moyen de commande est configuré pour que le fonctionnement du nébuliseur soit uniquement possible qu'une fois qu'un nombre prédéterminé de composants amovibles (5, 7, 9, 13) sont détectés comme étant fixés au nébuliseur (1).

7. Nébuliseur (1) selon la revendication 1, dans lequel le support de données (9a) stocke une valeur de décompte relative à l'utilisation de l'ensemble grille (9), et dans lequel le moyen de commande est adapté pour empêcher une grille (9b) d'être utilisée dans le nébuliseur après un nombre d'utilisations prédéterminé.

8. Nébuliseur (1) selon l'une quelconque des revendications précédentes, dans lequel le support de données (5a, 7a, 9a) et le lecteur de données (11) comprennent un système d'étiquetage d'identification par radiofréquence (RFID).

9. Nébuliseur (1) selon la revendication 8, dans lequel le support de données (sua, 7a, 9a) comprend une étiquette RFID et le lecteur de données (11) comprend une antenne.

10. Nébuliseur (1) selon la revendication 8 ou 9, dans lequel la plage du système d'étiquetage RFID est restreinte pour que l'antenne (11) soit appropriée pour communiquer avec des étiquettes RFID (5a, 7a, 9a) montées dans des composants amovibles (5, 7, 9, 13) fixés au nébuliseur.

11. Nébuliseur (1) selon l'une quelconque des revendications 2 à 9, dans lequel l'au moins un composant amovible (5, 7, 9, 13) supplémentaire est l'un d'un embout buccal (5), d'un ensemble piston (7) ou d'une chambre de médicament (13).

12. Ensemble grille (9) pour une utilisation dans un nébuliseur, l'ensemble grille (9) comprenant un support de données (9a) pour communiquer, en utilisation, avec un lecteur de données (11) fourni dans le nébuliseur ;
dans lequel le support de données (9a) est adapté pour stocker des informations de commande du fonctionnement du nébuliseur.

13. Ensemble grille (9) selon la revendication 12, dans lequel le support de données (9a) est adapté pour stocker des informations pour empêcher une grille (9b) d'être utilisée dans un nébuliseur après un nombre d'utilisations prédéterminé.

14. Ensemble grille (9) selon la revendication 13, dans lequel le support de données (9a) stocke une valeur de décompte, la valeur de décompte étant incrémentée ou décrémentée après chaque utilisation de la grille.

15. Procédé d'exploitation d'un nébuliseur (1), le procédé comprenant les étapes de :
réception d'informations en provenance d'un support de données (5a, 7a, 9a) associées à un composant amovible (5, 7, 9, 13) du nébuliseur ; et
commande du fonctionnement du nébuliseur d'après les informations reçues en provenance du support de données (sua, 7a, 9a) ;
dans lequel le composant amovible comprend un ensemble grille.
